**Europäisches Patentamt**

**European Patent Office**

**Office européen des brevets**

(19)

(11) Publication number: **0 111 456**
**B1**

(12)

# EUROPEAN PATENT SPECIFICATION

(45) Date of publication of patent specification: **12.07.89**

(21) Application number: **83810581.5**

(22) Date of filing: **09.12.83**

(51) Int. Cl.⁴: **A 61 K 31/495,**
**A 61 K 31/505, A 61 K 31/62**
**// (A61K31/505, 31:495,**
**31:165),(A61K31/495, 31:19,**
**31:165),(A61K31/495, 31:165)**

(54) Analgesic combinations.

(30) Priority: **09.12.82 US 448290**

(43) Date of publication of application:
**20.06.84 Bulletin 84/25**

(45) Publication of the grant of the patent:
**12.07.89 Bulletin 89/28**

(84) Designated Contracting States:
**AT BE CH DE FR GB IT LI LU NL SE**

(56) References cited:
**UNLISTED DRUGS, vol. 28, no. 4, April 1976,**
**page 53, Chatham, New Jersey, US**
**UNLISTED DRUGS, vol. 25, no. 6, June 1973,**
**page 99, Chatham, New Jersey, US**
**UNLISTED DRUGS, vol. 25, no. 9, September**
**1973, page 141, Chatham, New Jersey, US**
**UNLISTED DRUGS, vol.19, no. 1, page 12,**
**January 1967, Chatham, New Jersey, US**
**UNLISTED DRUGS, vol. 15, no. 12, page 95,**
**December 1963, Chatham, New Jersey, US**
**CHEMICAL ABSTRACTS, vol. 81, no. 5, August**
**5, 1974, page 45, column 2, abstract 21095x,**
**Columbus, Ohio, US. K.V. KUZINA et al.:**
**"Interaction of analgesics and tranquilizers"**

(73) Proprietor: **Cooper, Stephen A. Dr.**
**1016 Pleasant Road**
**Bryn Mawr, Pa. 19010 (US)**

(72) Inventor: **Cooper, Stephen A. Dr.**
**1016 Pleasant Road**
**Bryn Mawr, Pa. 19010 (US)**

(74) Representative: **Shaw, Laurence Patent &**
**Trademark Agent**
**George House George Road**
**Birmingham B15 1PG (GB)**

# EP 0 111 456 B1

**Description**

The present invention relates to pharmaceutical compositions active as analgesics.

Many individual agents and combinations are known for alleviating the symptoms of pain. The quest for agents and combinations which will optimise the therapeutic effect and minimise undesired side effects continues in an attempt to find improved treatments.

1 - (p - chlorobenzhydryl) - 4 - (2 - (2 - hydroxyethoxy)ethyl)diethylene - diamine (hereinafter hydroxyzine) and salts thereof are known to be effective tranquilisers.

It is known from CA No. 5 (1974), No. 21095X that a composition comprising Aspirin (registered trade mark) and a minor amount of hydroxyzine can have a synergistic effect. The present invention is based on the realisation that the coadministration of hydroxyzine and a range of analgesics in defined proportions can have a beneficial synergistic effect. The agents are acetaminophen and/or one or more non-steroidal anti-inflammatory/analgesic agents (also known as NSAIDs). The invention is practised by administration adapted to achieve absorption through the alimentary canal, i.e. by oral or rectal administration, desirably by oral administration.

Accordingly, one aspect of the invention provides a synergistic composition in unit dosage form for oral or rectal administration to exert an analgesic effect, the composition comprising:

(a) one or more non-steroidal anti-inflammatory agent and/or acetaminophen; and

(b) hydroxyzine or a pharmaceutical salt thereof characterised in that component (a) is a non-steroidal anti-inflammatory agent other than Aspirin and/or acetaminophen and in that the content of component (b) is in the range of 25 to 100 mg and is in sufficient proportion in relation to component (a) to synergise the analgesic effect of component (a).

Active agent (b) is known and can be prepared e.g. as described in USP 2,899,426.

Any of a wide variety of non-steroidal, anti-inflammatory agents may be used as active agents (a). Useful materials include sodium 5 - (4 - chlorobenzoyl) - 1,4 - dimethyl - 1H - pyrrole - 2 - acetate dihydrate (generically referred to as zomepirac sodium); 4 - hydroxy - 2 - methyl - N - (2 - pyridyl) - 2H - 1,2 - benzothiazine - 3 - carboxamide - 1,1 - dioxide (generic name Piroxicam); 2',4' - difluoro - 4 - hydroxy - 3 - biphenylcarboxylic acid (generic name Diflunisal); 1 - isopropyl - 7 - methyl - 4 - phenyl - 2 (1H) - quinazolinone (generic name Proquazone); and arylacetic acid or arylpropionic acid compounds including the non-toxic therapeutically acceptable salts thereof, e.g. the sodium, potassium or calcium salts. Examples of useful arylacetic acid and arylpropionic acid compounds include 2 - (p - isobutylphenyl)propionic acid (generic name Ibuprofen); alpha - methyl - 4 - (2 - thienylcarbonyl) benzene acetic acid (generic name Suprofen); 4,5 - diphenyl - 2 - oxazole propionic acid (generic name Oxaprozin); rac - 6 - chloro - alpha - methylcarbazole - 2 - acetic acid (generic name Carprofen); 2 - (3 - phenyloxy-phenyl)propionic acid, particularly the calcium salt dihydrate thereof (these compounds being referred to generically as Fenoprofen and Fenoprofen calcium); 2 - (6 - methoxy - 2 - naphthyl) propionic acid (generic name Naproxen; the generic name of the sodium salt is Naproxen sodium); 4 - (1,3 - dihydro - 1 - oxo - 2H - isoindol - 2 - yl) - α - methylbenzene acetic acid (generic name Indoprofen); 2 - (3 - benzoylphenyl) propionic acid (generic name Ketoprofen); and 2 - (2 - fluoro - 4 - biphenylyl) propionic acid (generic name Flurbiprofen). 1,5 - (4 - Methylbenzoyl) - 1H - pyrrole - 2 - acetic acid (generic name Tolmetin). All of the aforementioned non-steroidal, anti-inflammatory agents and their preparation are known. For example the synthesis of zomerpirac sodium, fenoprofen, indoprofen and ibuprofen is described in U.S. 4,242,519, U.S. 3,752,826, U.S. 3,228,831 and U.S. 3,385,886.

Acetaminophen and its preparation are also well known.

It is preferred that active agents a) and b) are the sole active analgesic constituents in the pharmaceutical combinations according to the invention. Preferred as active agent b) is a non-steroidal anti-inflammatory agent in particular one or more of those listed above.

It is also preferred that hydroxyzine be employed in the form of its hydrochloride or pamoate salt.

The compositions according to the invention suitably contain pharmaceutically acceptable carriers or diluents which are admixed with the active agents and are conventional and well known in the art.

The compositions of this invention may be adapted for oral administration and administration through the rectum. Forms suitable for oral administration are for example tablets, dispersible powders, granules, capsules, syrups, elixirs or suspensions. Compositions for oral use contain one or more conventional adjuvants, such as sweetening agents, flavoring agents, coloring agents and preserving agents, in order to provide an elegant and palatable preparation. Tablets may contain the active ingredients in admixture with conventional pharmaceutically acceptable excipients, e.g. inert diluents, such as calcium carbonate, sodium carbonate, lactose, and talc, granulating and disintegrating agents, e.g. starch and alginic acid, binding agents, e.g. starch gelatin and acacia, and lubricating agents, e.g. magnesium stearate, stearic acid and talc. The tablets may be uncoated or coated by known techniques to delay distintegration and adsorption in the gastrointestinal tract and thereby provide a sustained action over a longer period. Similarly, suspensions, syrups and elixirs may contain the active ingredients in admixture with any of the conventional excipients utilized in the preparation of such compositions, e.g. suspending agents such as methyl-cellulose, tragacanth and sodium alginate; wetting agents such as lecithin, polyoxyethylene stearate and polyoxyethylene sorbitan monooleate; and preservatives such as ethyl p-hydroxybenzoate. Capsules may contain the active ingredients alone or admixed with an inert solid diluent, e.g. calcium

2

carbonate, calcium phosphate and kaolin. These pharmaceutical compositions may contain up to about 90% of the active ingredients in combination with the carrier or adjuvant. Preferably the compositions are put up in unit dosage form particularly in unit dosage form for oral administration. Such forms may contain the active ingredients separately, e.g. in separate layers in a layer or mantle tablet or in split capsules. Oral administration is preferred.

A further aspect of the invention concerns a process for the production of a pharmaceutical composition as stated above which comprises formulating active agent a) with active agent b) as stated above and optionally putting up the preparation in unit dosage form.

A clinical test was conducted involving subjects who were outpatients undergoing the surgical removal of impacted teeth. After surgery was completed, the patients were given one dose of a medication and a questionnaire. They were asked to take the medication when their post-operative pain reached moderate to severe intensity. They were instructed to record their starting pain level in numerical fashion, i.e., moderate (2) or severe (3) and then at each hour for the next four hours to record their pain intensity as severe (3), moderate (2), slight (1), or none (0); and their relief from the starting pain as complete (4), a lot (3), some (2), a little (1) or none (0). The procedure employed is state-of-the-art methodology and is described in more detail in an article entitled "A Model to Evaluate Mild Analgesics in Oral Surgery Out-patients", S. A. Cooper and W. T. Beaver, Clinical Pharmacology and Therapeutics, Vol., 20, Number 2, pp. 241—250, August, 1976.

In the test, the patients were given a placebo, 600 milligrams of acetaminophen, 100 milligrams of Hydroxyzine pamoate, or a mixture of 100 milligrams of Hydroxyzine pamoate and 600 milligrams of acetaminophen.

The results of the test are recorded in Table 1 below. Pain intensity difference scores were derived by subtracting the pain level at each hour after ingestion from the intensity at the time of initial administration. Hence if the patient's pain was judged to be "moderate" (value of 2) at the start of the test and the patient judged his pain to be "slight" at hours one, two, three and four (assigned value of 1), the pain intensity difference score would be 4, that is 2 minus 1 or plus 1 at each of the four hourly measuring points. Pain relief scores for each pain estimate were assigned according to patient's hourly estimate of relief. Accordingly, if the patient recorded a little relief at hour one into the test (a score of 1) and some relief at hour two (a score of 2) and a lot of relief at hours three and four (score 3 for each hour) his pain relief score would be 9.

TABLE 1

|  | Placebo | Acetaminophen 600 mg | Hydroxyzine pamoate 100 mg | Hydroxyzine 100 mg+ acetaminophen 600 mg |
|---|---|---|---|---|
| Number of patients | 6 | 7 | 4 | 6 |
| Mean sum pain intensity difference score | −0.17 | 3.14 | −0.50 | 4.50 |
| Mean sum pain relief score | 4.00 | 8.14 | 3.00 | 10.83 |

In a second test series, peridontal patients, after completion of surgery, were tested using the above procedures except that they were requested to record pain intensity and pain relief levels at 30 minutes, and then at each hour for the next 4 hours. The test scores are therefore the sum of five readings rather than 4 readings as in the above test. In this test the patients were given 100 mg of Hydroxyzine pamoate, 400 mg ibuprofen or a mixture of 100 mg hydroxyzine pamoate with 400 mg of ibuprofen.

Results

## TABLE 2

|  | Hydroxyzine pamoate 100 mg | Ibuprofen 400 mg | Combined |
|---|---|---|---|
| Number of patients | 12 | 13 | 11 |
| Mean sum pain intensity difference score | 0.33 | 6.69 | 7.73 |
| Mean sum pain relief score | 2.17 | 13.54 | 14.73 |

The data obtained from this second test when plotted showed at the end of 4 hours that the line of the curve for the combination was continuing essentially horizontally whereas the line for Ibuprofen alone was declining at an angle of about 20°.

In a third test series, oral surgery patients, after completion of surgery, were tested using the same procedures except that they were requested to record pain intensity and pain relief levels at 30 minutes and then at each hour for the next six hours. The test scores are therefore the sum of seven readings rather than four readings as in the first test. In the tests, the patients were given a placebo, 200 milligrams of fenoprofen calcium, 100 mg of hydroxyzine pamoate or a mixture of 100 mg of hydroxyzine pamoate with 200 mg of fenoprofen calcium. The test data are shown in Table 3 below.

## TABLE 3

|  | Placebo | Hydroxyzine pamoate 100 mg | Fenoprofen calcium 200 mg | Hydroxyzine pamoate 100 mg+fenoprofen calcium 200 mg |
|---|---|---|---|---|
| Number of patients | 9 | 11 | 11 | 10 |
| Mean sum pain intensity difference score | 1.56 | 1.50 | 3.55 | 9.60 |
| Mean sum pain relief score | 8.33 | 3.25 | 12.00 | 18.40 |

Thus the above tests indicate a synergism of the potentiating kind for the combination of active agent a) with an active agent b).

They also confirm literature studies indicating that hydroxyzine alone is inactive orally as an analgesic.

The combination is thus indicated for use in alleviating the symptoms of pain.

Accordingly the invention further provides a method of alleviating the symptoms of pain in a subject in need of such treatment, which method comprises concomitantly administering to said subject an effective amount of an active agent a) and an active agent b) as stated above.

It has also been determined that co-administration of active agents a) and b) does not possess the nausea-causing properties normally associated with analgesic combinations. Co-administration of active agents a) and b) also exhibits anti-emetic and anti-histaminic effects.

The therapeutic dose differs with the kind of pharmaceutic process, severity of the condition, administration schedule and other known factors when co-administration is envisaged, and doses typically continued until the condition causing the pain is ameliorated. Usually, the NSAID or acetaminophen will be co-administered with a pain relieving potentiating effective amount of hydroxyzine (or its salts), in a total combined pain relieving effective amount, in doses given 3 to 6 times a day as needed to relieve pain. In general, at least 25 mg of hydroxyzine and its salts will be administered per dose, preferably at least 50 mg and typically at least 70 mg. A dose for each administration of hydroxyzine and its salts may range from 25 mg to 120 mg, more usually 25 mg to 100 mg, preferably 50 mg to 100 mg, and typically 70 mg to 100 mg. The amount of the non-steroid anti-inflammatory agent to be co-administered with the hydroxyzine will vary depending mainly on the particular agent desired. In general, it is desirable to employ at least an amount of the NSAID (or acetaminophen when it may be used) that would by itself be minimally effective clinically in the adult human to produce analgesia.

The particularly preferred NSAIDs are those classed as arylacetic and arylpropionic acids (and the pharmaceutically acceptable salts) including, for example, ibuprofen, oxaprozin, carprofen, fenoprofen or fenoprofen calcium, naproxin or naproxin sodium, indoprofen, ketoprofen, flurbiprofen and tolmetin or tolmetin sodium. Also of particular interest are the NSAIDs proquazone, zomepirac, piroxicam and diflunisal.

In Table I, below, there are given various NSAIDs of particular interest along with general (a), preferred (b) and in some cases more preferred (c) milligram dose ranges for each administration (Column A); the weight ratio thereto of hydroxyzine when the hydroxyzine dose to be administered is within the range of 25 to 100 mg (Column B); and the weight ratio thereto of hydroxyzine when the hydroxyzine does to be administered is within the range of 50 to 100 mg (Column C).

Suitable doses for acetaminophen are from 200—1,300 mg, preferably 300—650 mg, leading to the same weight ratios with hydroxyzine (25 to 100 mg range and 50 to 100 mg range) as indicated for aspirin in Table I.

The limits of weight ratio ranges of hydroxyzine for the hydroxyzine dose ranges of 25 to 120 mg and 70 to 100 mg, to the dose ranges for NSAIDs given in Table I may be readily calculated from the dose ranges given in Column A of Table I for the particular NSAID (as well as from the above information for acetaminophen), and such weight ratio ranges are also deemed disclosed herein. It will be evident that the weight ratio ranges of hydroxyzine to the NSAID or acetaminophen as indicated above may be used to formulate pharmaceutical compositions in accordance with the invention, provided that a NSAID or acetaminophen pain relieving potentiating effective amount of hydroxyzine is included.

|  | Column A | Column B | Column C |
|---|---|---|---|
|  | NSAID Dose range | Weight ratio hydroxyzine range at 25—100 mg to NSAID dose range | Weight ratio hydroxyzine range at 50—100 mg to NSAID dose range |
| NSAID Ibuprofen | a) 100—800<br>b) 150—600<br>c) 200—400 | 1:32 to 1:1<br>1:24 to 1:1.5<br>1:16 to 1:2 | 1:16 to 1:1<br>1:12 to 1:1.5<br>1:8 to 1:2 |
| Oxaprozin | a) 300—1200<br>b) 400—800 | 1:48 to 1:3<br>1:32 to 1:4 | 1:24 to 1:3<br>1:16 to 1:4 |
| Carprofen | a) 50—400<br>b) 100—300 | 1:16 to 1:0.5<br>1:12 to 1:1 | 1:8 to 1:0.5<br>1:6 to 1:1 |
| Fenoprofen or Fenoprofen calcium | a) 50—600<br>b) 100—400<br>c) 150—300 | 1:24 to 1:0.5<br>1:16 to 1:1<br>1:12 to 1:1.5 | 1:12 to 1:0.5<br>1:8 to 1:1<br>1:6 to 1:1.5 |
| Naproxen or Naproxen sodium | a) 100—600<br>b) 200—400 | 1:24 to 1:1<br>1:16 to 1:2 | 1:12 to 1:1<br>1:8 to 1:2 |
| Indoprofen | a) 25—200<br>b) 50—150 | 1:8 to 1:0.25<br>1:6 to 1:0.5 | 1:4 to 1:0.25<br>1:3 to 1:0.5 |
| Ketoprofen | a) 25—300<br>b) 50—200 | 1:12 to 1:0.25<br>1:8 to 1:0.5 | 1:6 to 1:0.25<br>1:4 to 1:0.5 |
| Flurbiprofen | a) 15—200<br>b) 25—150 | 1:8 to 1:0.15<br>1:6 to 1:0.25 | 1:4 to 1:0.15<br>1:3 to 1:0.25 |
| Zomepirac | a) 15—200<br>b) 25—100 | 1:4 to 1:0.15<br>1:4 to 1:0.25 | 1:2 to 1:0.15<br>1:2 to 1:0.25 |
| Piroxicam | a) 10—50<br>b) 15—37.5 | 1:2 to 1:0.1<br>1:1.5 to 1:0.15 | 1:1 to 1:0.1<br>1:0.75 to 1:0.15 |
| Diflunisal | a) 125—1000<br>b) 250—750 | 1:40 to 1:1.25<br>1:30 to 1:2.5 | 1:20 to 1:1.25<br>1:15 to 1:2.5 |
| Proquazone | a) 60—300<br>b) 75—150 | 1:2 to 1:0.6<br>1:6 to 1:0.75 | 1:6 to 1:0.6<br>1:3 to 1:0.75 |
| Aspirin | a) 200—1300<br>b) 300—650 | 1:52 to 1:2<br>1:26 to 1:3 | 1:26 to 1:2<br>1:13 to 1:3 |
| Tolmetin or Tolmetin sodium | a) 300—800<br>b) 400—600 | 1:32 to 1:3<br>1:24 to 1:4 | 1:16 to 1:3<br>1:12 to 1:4 |

Conveniently the active agents will be administered in fixed combinations having e.g. the individual dosages outlined above and in unit dosage form.

They can be administered e.g. in sustained release form or in divided doses 2 to 4 times a day or as indicated by the condition to be treated e.g. 3 to 6 times as needed to relieve pain.

The active agents a) and b) can also be administered e.g. in the indicated dosages individually and concomitantly.

The invention therefore also provides a pack comprising separately a plurality of dosage units of each of active agents a) and b) together with instructions for their concomitant administration.

The following Examples are illustrative of compositions for use in the invention.

Example A

A No. 1 capsule containing the ingredients indicated below may be prepared by conventional techniques and administered at a dose of 1 or 2 capsules 4 to 6 times a day to relieve pain.

| Ingredient | Weight (mg) |
| --- | --- |
| Ibuprofen | 200 |
| Hydroxyzine pamoate | 50 |
| Corn starch | 150 |
| Magnesium stearate | 2 |

Example B

A tablet containing the ingredients indicated below may be prepared by conventional techniques and administered at a dose of 1 or 2 tablets 3 to 5 times a day to relieve pain.

| Ingredient | Weight (mg) |
| --- | --- |
| Proquazone (micronized) | 75 |
| Hydroxyzine hydrochloride | 25, 50 or 75 |
| Lactose powder | 279.2 |
| Alginic acid | 90 |
| Yellow iron oxide (T-1624) | 1.5 |
| Celloidal silicon dioxide | 13 |
| Pluronic F-68 | 1.3 |
| Poridone | 10 |
| Stearic acid | 5 |

Using a single dose tablet containing 75 mg of hydroxyzine hydrochloride and formulated as in Example B (75 mg of proquazone), and administered in a clinical test as described above, indicated after 6 hours a clear potentiation of the pain relieving effects of proquazone by the hydroxyzine.

**Claims**

1. A synergistic composition in unit dosage form for oral or rectal administration to exert an analgesic effect, the composition comprising:

(a) one or more non-steroidal anti-inflammatory agent and/or acetaminophen; and

(b) hydroxyzine or a pharmaceutical salt thereof characterised in that component (a) is a non-steroidal anti-inflammatory agent other than Aspirin and/or acetaminophen and in that the content of component (b) is in the range of 25 to 100 mg and is in sufficient proportion in relation to component (a) to synergise the analgesic effect of component (a).

2. A synergistic composition according to Claim 1 characterised in that the unit dosage form comprises 100 mg to 800 mg of ibuprofen and when 25 to 100 mg of hydroxyzine is present the weight ratio of hydroxyzine:ibuprofen is 1:32 to 1:1 and when 50 to 100 mg of hydroxyzine is present the hydroxyzine:ibuprofen weight ratio is 1:16 to 1:1.

3. A synergistic composition according to Claim 2 characterised in that the unit dosage form comprises 150 mg to 600 mg of ibuprofen and when 25 to 100 mg of hydroxyzine is present the weight ratio of hydoxyzine:ibuprofen is 1:24 to 1:1.5 and when 50 to 100 mg of hydroxyzine is present the hydroxyzine:ibuprofen weight ratio is 1:12 to 1:1.5.

4. A synergistic composition according to Claim 2 or 3 characterised in that the unit dosage form comprises 200 mg to 400 mg of ibuprofen and when 25 to 100 mg of hydroxyzine is present the weight ratio of hydroxyzine:ibuprofen is 1:16 to 1:2 and when 50 to 100 mg of hydroxyzine is present the weight ratio of hydroxyzine:ibuprofen is 1:8 to 1:2.

5. A synergistic composition according to Claim 1 characterised in that the unit dosage form comprises 300 mg to 1200 mg of oxaprozin and when 25 to 100 mg of hydroxyzine is present the weight ratio of hydroxyzine:oxaprozin is 1:48 to 1:3 and when 50 to 100 mg of hydroxyzine is present the hydroxyzine:oxaprozin weight ratio is 1:24 to 1:3.

6. A synergistic composition according to Claim 5 characterised in that the unit dosage form comprises 400 mg to 800 mg of oxaprozin and when 25 to 100 mg of hydroxyzine is present the weight ratio of hydroxyzine:oxaprozin is 1:32 to 1:4 and when 50 to 100 mg of hydroxyzine is present the hydroxyzine:oxaprozin weight ratio is 1:16 to 1:4.

7. A synergistic composition according to Claim 1 characterized in that the unit dosage form comprises 50 mg to 400 mg of carprofen and when 25 to 100 mg of hydroxyzine is present the weight ratio of hydroxyzine:carprofen is 1:16 to 1:0.5 and when 50 to 100 mg of hydroxyzine is present the hydroxyzine:carprofen weight ratio is 1:8 to 1:0.5.

8. A synergistic composition according to Claim 7 characterised in that the unit dosage form comprises 100 mg to 300 mg of carprofen and when 25 to 100 mg of hydroxyzine is present the weight ratio of hydroxyzine:carprofen is 1:12 to 1:1 and when 50 to 100 mg of hydroxyzine is present the hydroxyzine:carprofen weight ratio is 1:6 to 1:1.

9. A synergistic composition according to Claim 1 characterised in that the unit dosage form comprises 50 mg to 600 mg of fenoprofen or a salt thereof and when 25 to 100 mg of hydroxyzine is present the weight ratio of hydroxyzine:fenoprofen or salt thereof is 1:24 to 1:0.5.

10. A synergistic composition according to Claim 9 characterised in that the unit dosage form comprises 100 mg to 400 mg of fenoprofen or a salt thereof and when 25 to 100 mg of hydroxyzine is present the weight ratio of hydroxyzine:fenoprofen or salt thereof is 1:16 to 1:1 and when 50 to 100 mg of hydroxyzine is present the weight ratio of hydroxyzine:fenoprofen or salt thereof is 1:8 to 1:1.

11. A synergistic composition according to Claim 9 or 10 characterised in that the unit dosage form comprises 150 mg to 300 mg of fenoprofen or a salt thereof and when 25 to 100 mg of hydroxyzine is present the weight ratio of hydroxyzine:fenoprofen or salt thereof is 1:12 to 1:1.5 and when 50 to 100 mg of hydroxyzine is present the weight ratio of hydroxyzine:fenoprofen or salt thereof is 1:6 to 1:1.5.

12. A synergistic composition according to Claim 1 characterised in that the unit dosage form comprises 100 mg to 600 mg of naproxen or a salt thereof and when 25 to 100 mg of hydroxyzine is present the weight ratio of hydroxyzine:naproxen or salt thereof is 1:24 to 1:1 and when 50 to 100 mg of hydroxyzine is present the weight ratio of hydroxyzine:naproxen or salt thereof is 1:12 to 1:1.

13. A synergistic composition according to Claim 12 characterised in that the unit dosage form comprises 200 mg to 400 mg of naproxen or a salt thereof and when 25 to 100 mg of hydroxyzine is present the weight ratio of hydroxyzine:naproxen or salt thereof is 1:16 to 1:1.2 and when 50 to 100 mg of hydroxyzine is present the weight ratio of hydroxyzine:naproxen or salt thereof is 1:18 to 1:12.

14. A synergistic composition according to Claim 1 characterised in that the unit dosage form comprises 25 mg to 200 mg of indoprofen and when 25 to 100 mg of hydroxyzine is present the weight ratio of hydroxyzine:indoprofen is 1:8 to 0.25 and when 50 to 100 mg of hydroxyzine is present the hydroxyzine:indoprofen weight ratio is 1:4 to 1:1.

15. A synergistic composition according to Claim 14 characterised in that the unit dosage form comprises 50 mg to 150 mg of indoprofen and when 25 to 100 mg of hydroxyzine is present the weight ratio of hydroxyzine:indoprofen is 1:6 to 1:0.5 and when 50 to 100 mg of hydroxyzine is present the hydroxyzine:indoprofen weight ratio is 1:3 to 1:0.5.

16. A synergistic composition according to Claim 1 characterised in that the unit dosage form comprises 25 mg to 300 mg of ketoprofen and when 25 to 100 mg of hydroxyzine is present the weight ratio of hydroxyzine:ketoprofen is 1:12 to 1:0.25 and when 50 to 100 mg of hydroxyzine is present the hydroxyzine:ketoprofen weight ratio is 1:6 to 1:0.25.

17. A synergistic composition according to Claim 16 characterised in that the unit dosage form comprises 50 mg to 200 mg of ketoprofen and when 25 to 100 mg of hydroxyzine:ketoprofen is 1:8 to 1:0.5 and when 50 to 100 mg of hydroxyzine is present the hydroxyzine:ketoprofen weight ratio is 1:4 to 1:0.5.

18. A synergistic composition according to Claim 1 characterised in that the unit dosage form comprises 15 mg to 200 mg of flurbiprofen and when 25 to 100 mg of hydroxyzine is present the weight ratio of hydroxyzine:flurbiprofen is 1:8 to 1:15 and when 50 to 100 mg of hydroxyzine is present the hydroxyzine:flurbiprofen weight ratio is 1:4 to 1.15.

19. A synergistic composition according to Claim 18 characterised in that the unit dosage form comprises 25 mg to 150 mg of flurbiprofen and when 25 to 100 mg of hydroxyzine is present the weight ratio of hydoxyzine:flurbiprofen is 1:6 to 1:0.25 and when 50 to 100 mg of hydroxyzine is present the hydroxyzine:flurbiprofen weight ratio is 1:3 to 1:0.25.

20. A synergistic composition according to Claim 1 characterised in that the unit dosage form comprises 15 mg to 200 mg of zomepirac and when 25 to 100 mg of hydroxyzine is present the weight ratio of hydroxyzine:zomepirac is 1:4 to 1:0.15 and when 50 to 100 mg of hydroxyzine is present the hydroxyzine:zomepirac weight ratio is 1:2 to 1:1.15.

21. A synergistic composition according to Claim 20 characterised in that the unit dosage form comprises 25 mg to 100 mg of zomepirac and when 25 to 100 mg of hydroxyzine is present the weight ratio of hydroxyzine:zomepirac is 1:4 to 1:0.15 and when 50 to 100 mg of hydroxyzine is present the hydroxyzine:zomepirac weight ratio is 1:2 to 1:0.25.

22. A synergistic composition according to Claim 1 characterised in that the unit dosage form comprises 10 mg to 50 mg of piroxicam and when 25 to 100 mg of hydroxyzine is present the weight ratio of hydroxyzine:piroxicam is 1:2 to 1:0.1 and when 50 to 100 mg of hydroxyzine is present the hydroxyzine:piroxicam weight ratio is 1:1 to 1:0.1.

23. A synergistic composition according to Claim 22 characterised in that the unit dosage form comprises 15 mg to 37.5 mg of piroxicam and when 25 to 100 mg of hydroxyzine is present the weight ratio of hydroxyzine:piroxicam is 1:1.5 to 1:0.15 and when 50 to 100 mg of hydroxyzine is present the hydroxyzine:piroxicam weight ratio is 1:0.75 to 1:0.15.

24. A synergistic composition according to Claim 1 characterised in that the unit dosage form comprises 125 mg to 1000 mg of diflunisal and when 25 to 100 mg of hydroxyzine is present the weight ratio of hydroxyzine:diflunisal is 1:40 to 1:25 and when 50 to 100 mg of hydroxyzine is present the hydroxyzine:diflunisal weight ratio is 1:20 to 1:1.25.

25. A synergistic composition according to Claim 24 characterised in that the unit dosage form comprises 250 mg to 750 mg of diflunisal and when 25 to 100 mg of hydroxyzine is present the weight ratio of hydroxyzine:diflunisal is 1:30 to 1:2.5 and when 50 to 100 mg of hydroxyzine is present the hydroxyzine:diflunisal weight ratio is 1:15 to 1:2.5.

26. A synergistic composition according to Claim 1 characterised in that the unit dosage form comprises 60 mg to 300 mg of proquazone and when 25 to 100 mg of hydroxyzine is present the weight ratio of hydroxyzine:proquazone is 1:2 to 1:0.6 and when 50 to 100 mg of hydroxyzine is present the hydroxyzine:proquazone weight ratio is 1:6 to 1:0.6.

27. A synergistic composition according to Claim 26 characterised in that the unit dosage form comprises 75 mg to 150 mg of proquazone and when 25 to 100 mg of hydroxyzine is present the weight ratio of hydroxyzine:proquazone is 1:6 to 1:0.75 and when 50 to 100 mg of hydroxyzine is present the hydroxyzine:proquazone weight ratio is 1:3 to 1:0.75.

28. A synergistic composition according to Claim 1 characterised in that the unit dosage form comprises 200 mg to 1300 mg of acetaminophen and when 25 to 100 mg of hydroxyzine is present the weight ratio of hydroxyzine:acetaminophen is 1.52 to 1:2 and when 50 to 100 mg of hydroxyzine is present the hydroxyzine:acetaminophen weight ratio is 1:26 to 1:2.

29. A synergistic composition according to Claim 28 characterised in that the unit dosage form comprises 300 mg to 650 mg of acetaminophen and when 25 to 100 mg of hydroxyzine is present the weight ratio of hydroxyzine:acetaminophen is 1:26 to 1:2 and when 50 to 100 mg of hydroxyzine is present the hydroxyzine:acetaminophen weight ratio is 1:13 to 1:3.

30. A synergistic composition according to Claim 1 characterised in that the unit dosage form comprises 300 mg to 800 mg of tolmetin or a salt thereof and when 25 to 100 mg of hydroxyzine is present the weight ratio of hydroxyzine:tolmetin or salt thereof is 1:32 to 1:3 and when 50 to 100 mg of hydroxyzine is present the weight ratio of hydroxyzine:tolmetin or salt thereof is 1:16 to 1:3.

31. A synergistic composition according to Claim 30 characterised in that the unit dosage form comprises 400 mg to 600 mg of tolmetin or a salt thereof and when 25 to 100 mg of hydroxyzine is present the weight ratio of hydroxyzine:tolmetin or salt thereof is 1:24 to 1:4 and when 50 to 100 mg of hydroxyzine is present the weight ratio of hydroxyzine:tolmetin or salt thereof is 1:12 to 1:4.

**Patentansprüche**

1. Synergistische Zusammensetzung in Einzeldosis-Form für die orale oder rektale Verabreichung zur Ausübung einer schmerzstillenden Wirkung. Die Zusammensetzung besteht aus:
(a) Einem oder mehreren nichtsteroiden entzündungshemmenden Mitteln, bzw. Acetaminophen und
(b) Hydroxyzin und einem pharmazeutischen Salz desselben, dadurch gekennzeichnet, daß die Komponente (a) ein nichtsteroides entzündungshemmendes Mittel außer Asprin ist bzw. Acetaminophen und darin, daß der Gehalt von Komponente (b) im Bereich von 25 bis 100 mg liegt und im Verhältnis in bezug auf Komponente (a) zum Synergieren der schmerzstillenden Wirkung von Komponente (a) ausreicht.

2. Synergistische Zusammensetzung nach Anspruch 1, dadurch gekennzeichnet, daß die Einzeldosis-Form 100 bis 800 mg Ibuprofen enthält und daß bei einem Gehalt von 25 bis 100 mg Hydroxyzin das Gewichtsverhältnis von Hydroxyzin:Ibuprofen 1:32 bis 1:1 beträgt und bei einem Gehalt von 50 bis 100 mg Hydroxyzin das Gewichtsverhältnis von Hydroxyzin:Ibuprofen 1:16 bis 1:1 beträgt.

3. Synergistische Zusammensetzung nach Anspruch 2, dadurch gekennzeichnet, daß die Einzeldosis-Form 150 bis 600 mg Ibuprofen enthält und bei einem Gehalt von 25 bis 100 mg Hydroxyzin ein Gewichtsverhältnis von Hydroxyzin:Ibuprofen von 1:24 bis 1:1,5 entsteht und bei einem Gehalt von 50 bis 100 mg Hydroxyzin ein Gewichtsverhältnis von Hydroxyzin:Ibuprofen von 1:12 bis 1:1,5 entsteht.

4. Synergistische Zusammensetzung nach Anspruch 2 oder 3, dadurch gekennzeichnet, daß die Einzeldosis-Form 200 bis 400 mg Ibuprofen enthält und bei einem Gehalt von 25 bis 100 mg Hydroxyzin ein Gewichtsverhältnis von Hydroxyzin:Ibuprofen von 1:16 bis 1:2 entsteht und bei einem Gehalt von 50 bis 100 mg Hydroxyzin ein Gewichtsverhältnis von Hydroxyzin:Ibuprofen von 1:8 bis 1:2 entsteht.

5. Synergistische Zusammensetzung nach Anspruch 1, dadurch gekennzeichnet, daß die Einzeldosis-Form 300 bis 1200 mg Oxaprozin enthält und bei einem Gehalt von 25 bis 100 mg Hydroxyzin ein Gewichtsverhältnis von Hydroxyzin:Oxaprozin von 1:48 bis 1:3 entsteht und bei einem Gehalt von 50 bis 100 mg Hydroxyzin ein Gewichtsverhältnis von Hydroxyzin:Oxaprozin von 1:24 bis 1:3 entsteht.

6. Synergistische Zusammensetzung nach Anspruch 5, dadurch gekennzeichnet, daß die Einzeldosis-Form 400 bis 800 mg Oxaprozin enthält und bei einem Gehalt von 25 bis 100 mg Hydroxyzin ein Gewichtsverhältnis von Hydroxyzin:Oxaprozin von 1:32 bis 1:4 entsteht und bei einem Gehalt von 50 bis 100 mg Hydroxyzin ein Gewichtsverhältnis von Hydroxyzin:Oxaprozin von 1:16 bis 1:4 entsteht.

7. Synergistische Zusammensetzung nach Anspruch 1, dadurch gekennzeichnet, daß die Einzeldosis-Form 50 bis 400 mg Carprofen enthält und bei einem Gehalt von 25 bis 100 mg Hydroxyzin ein Gewichtsverhältnis von Hydroxyzin:Carprofen von 1:16 bis 1:0,5 entsteht und bei einem Gehalt von 50 bis 100 mg Hydroxyzin ein Gewichtsverhältnis von Hydroxyzin:Carprofen von 1:8 bis 1:0,5 entsteht.

8. Synergistische Zusammensetzung nach Anspruch 7, dadurch gekennzeichnet, daß die Einzeldosis-Form 100 bis 300 mg Carprofen enthält und bei einem Gehalt von 25 bis 100 mg Hydroxyzin ein Gewichtsverhältnis von Hydroxyzin:Carprofen von 1:12 bis 1:1 entsteht und bei einem Gehalt von 50 bis 100 mg Hydroxyzin ein Gewichtsverhältnis von Hydroxyzin:Carprofen von 1:6 bis 1:1 entsteht.

9. Synergistische Zusammensetzung nach Anspruch 1, dadurch gekennzeichnet, daß die Einzeldosis-Form 50 bis 600 mg Fenoprofen oder ein Salz desselben enthält und bei einem Gehalt von 25 bis 100 mg Hydroxyzin ein Gewichtsverhältnis von Hydroxyzin:Fenoprofen oder einem Salz desselben von 1:24 bis 1:0,5 entsteht.

10. Synergistische Zusammensetzung nach Anspruch 9, dadurch gekennzeichnet, daß die Einzeldosis-Form 100 bis 400 mg Fenoprofen oder ein Salz desselben enthält und bei einem Gehalt von 25 bis 100 mg Hydoxyzin ein Gewichtsverhältnis von Hydroxyzin:Fenoprofen oder einem Salz desselben von 1:16 bis 1:1 entsteht und bei einem Gehalt von 50 bis 100 mg Hydroxyzin ein Gewichtsverhältnis von Hydroxyzin:Fenoprofen oder ein Salz desselben von 1:8 bis 1:1 entsteht.

11. Synergistische Zusammensetzung nach Anspruch 9 oder 10, dadurch gekennzeichnet, daß die Einzeldosis-Form 150 bis 300 mg Fenoprofen oder ein Salz desselben enthält und bei einem Gehalt von 25 bis 100 mg Hydroxyzin ein Gewichtsverhältnis von Hydroxyzin:Fenoprofen oder ein Salz desselben von 1:12 bis 1:1,5 entsteht und bei einem Gehalt von 50 bis 100 mg Hydroxyzin das Gewichtsverhältnis von Hydroxyzin:Fenoprofen oder einem Salz desselben 1:6 bis 1:1,5 beträgt.

12. Synergistische Zusammensetzung nach Anspruch 1, dadurch gekennzeichnet, daß die Einzeldosis-Form 100 bis 600 mg Naproxen oder ein Salz desselben enthält und bei einem Gehalt von 25 bis 100 mg Hydroxyzin ein Gewichtsverhältnis von Hydoxyzin:Naproxen oder einem Salz desselben von 1:24 bis 1:1 entsteht und bei einem Gehalt von 50 bis 100 mg Hydroxyzin ein Gewichtsverhältnis von Hydroxyzin:Naproxen oder einem Salz desselben von 1:12 bis 1:1 entsteht.

13. Synergistische Zusammensetzung nach Anspruch 12, dadurch gekennzeichnet, daß die Einzeldosis-Form 200 bis 400 mg Naproxen oder ein Salz desselben enthält und bei einem Gehalt von 25 bis 100 mg Hydroxyzin ein Gewichtsverhältnis von Hydroxyzin:Naproxen oder einem Salz desselben von 1:16 bis 1:1,2 entsteht und bei einem Gehalt von 50 bis 100 mg Hydroxyzin ein Gewichtsverhältnis von Hydroxyzin:Naproxen oder einem Salz desselben von 1:18 bis 1:12 entsteht.

14. Synergistische Zusammensetzung nach Anspruch 1, dadurch gekennzeichnet, daß die Einzeldosis-Form 25 bis 200 mg Indoprofen enthält und bei einem Gehalt von 25 bis 100 mg Hydroxyzin ein Gewichtsverhältnis von Hydroxyzin:Indoprofen von 1:8 bis 1:0,25 entsteht und bei einem Gehalt von 50 bis 100 mg Hydroxyzin ein Gewichtsverhältnis von Hydroxyzin:Indoprofen von 1:4 bis 1:1 entsteht.

15. Synergistische Zusammensetzung nach Anspruch 14, dadurch gekennzeichnet, daß die Einzeldosis-Form 50 bis 150 mg Indoprofen enthält und bei einem Gehalt von 25 bis 100 mg Hydroxyzin ein Gewichtsverhältnis von Hydroxyzin:Indoprofen von 1:6 bis 1:0,5 entsteht und bei einem Gehalt von 50 bis 100 mg Hydroxyzin ein Gewichtsverhältnis von Hydroxyzin:Indoprofen von 1:3 bis 1:0,5 entsteht.

16. Synergistische Zusammensetzung nach Anspruch 1, dadurch gekennzeichnet, daß die Einzeldosis-Form 25 bis 300 mg Ketoprofen enthält und bei einem Gehalt von 25 bis 100 mg Hydroxyzin ein Gewichtsverhältnis von Hydroxyzin:Ketoprofen von 1:12 bis 1:0,25 entsteht und bei einem Gehalt von 50 bis 100 mg Hydroxyzin ein Gewichtsverhältnis von Hydroxyzin:Ketoprofen von 1:6 bis 1:0,25 entsteht.

17. Synergistische Zusammensetzung nach Anspruch 16, dadurch gekennzeichnet, daß die Einzeldosis-Form 50 bis 200 mg Ketoprofen enthält und bei einem Gehalt von 25 bis 100 mg Hydroxyzin ein Gewichtsverhältnis von Hydroxyzin:Ketoprofen von 1:8 bis 1:0,5 entsteht und bei einem Gehalt von 50 bis 100 mg Hydroxyzin ein Gewichtsverhältnis von Hydroxyzin:Ketoprofen von 1:4 bis 1:0,5 entsteht.

18. Synergistische Zusammensetzung nach Anspruch 1, dadurch gekennzeichnet, daß die Einzeldosis-Form 15 bis 200 mg Flurbiprofen enthält und bei einem Gehalt von 25 bis 100 mg Hydroxyzin ein Gewichtsverhältnis von Hydroxyzin:Flurbiprofen von 1:8 bis 1:15 entsteht und bei einem Gehalt von 50 bis 100 mg Hydroxyzin ein Gewichtsverhältnis von Hydroxyzin:Flurbiprofen von 1:4 bis 1:1.15 entsteht.

19. Synergistische Zusammensetzung nach Anspruch 18, dadurch gekennzeichnet, daß die Einzeldosis-Form 25 bis 150 mg Flurbiprofen enthält und bei einem Gehalt von 25 bis 100 mg Hydroxyzin ein Gewichtsverhältnis von Hydroxyzin:Flurbiprofen von 1:6 bis 1:0,25 entsteht und bei einem Gehalt von 50 bis 100 mg Hydroxyzin ein Gewichtsverhältnis von Hydroxyzin:Flurbiprofen von 1:3 bis 1:0,25 entsteht.

20. Synergistische Zusammensetzung nach Anspruch 1, dadurch gekennzeichnet, daß die Einzeldosis-Form 15 bis 200 mg Zomepirac enthält und bei einem Gehalt von 25 bis 100 mg Hydroxyzin ein Gewichtsverhältnis von Hydroxyzin:Zomepirac von 1:4 bis 1:0,15 entsteht und bei einem Gehalt von 50 bis 100 mg Hydroxyzin ein Gewichtsverhältnis von Hydroxyzin:Zomepirac von 1:2 bis 1:1.15 entsteht.

21. Synergistische Zusammensetzung nach Anspruch 20, dadurch gekennzeichnet, daß die Einzeldosis-Form 25 bis 100 mg Zomepirac enthält und bei einem Gehalt von 25 bis 100 mg Hydroxyzin ein Gewichtsverhältnis von Hydroxyzin:Zomepirac von 1:4 bis 1:0,15 entsteht und bei einem Gehalt von 50 bis 100 mg Hydroxyzin ein Gewichtsverhältnis von Hydroxyzin:Zomepirac von 1:1 bis 1:0,25 entsteht.

22. Synergistische Zusammensetzung nach Anspruch 1, dadurch gekennzeichnet, daß die Einzeldosis-Form 10 bis 50 mg Piroxicam enthält und bei einem Gehalt von 25 bis 100 mg Hydroxyzin ein Gewichtsverhältnis von Hydroxyzin:Piroxicam von 1:2 bis 1:0,1 entsteht und bei einem Gehalt von 50 bis 100 mg Hydroxyzin ein Gewichtsverhältnis von Hydroxyzin:Piroxicam von 1:1 bis 1:0,1 entsteht.

23. Synergistische Zusammensetzung nach Anspruch 22, dadurch gekennzeichnet, daß die Einzeldosis-Form 15 bis 37,5 mg Piroxicam enthält und bei einem Gehalt von 25 bis 100 mg Hydroxyzin ein Gewichtsverhältnis von Hydroxyzin:Piroxicam von 1:1,5 bis 1:0,15 entsteht und bei einem Gehalt von 50 bis 100 mg Hydroxyzin ein Gewichtsverhältnis von Hydroxyzin:Piroxicam von 1:0,75 bis 1:0,15 entsteht.

24. Synergistische Zusammensetzung nach Anspruch 1, dadurch gekennzeichnet, daß die Einzeldosis-Form 125 bis 1000 mg Diflunisal enthält und bei einem Gehalt von 25 bis 100 mg Hydroxyzin ein Gewichtsverhältnis von Hydroxyzin:Diflunisal von 1:40 bis 1:25 entsteht und bei einem Gehalt von 50 bis 100 mg Hydroxyzin ein Gewichtsverhältnis von Hydoxyzin:Diflunisal von 1:20 bis 1:1,25 entsteht.

25. Synergistische Zusammensetzung nach Anspruch 24, dadurch gekennzeichnet, daß die Einzeldosis-Form 250 bis 750 mg Diflunisal enthält und bei einem Gehalt von 25 bis 100 mg Hydroxyzin ein Gewichtsverhältnis von Hydroxyzin:Diflunisal von 1:30 bis 1:2,5 entsteht und bei einem Gehalt von 50 bis 100 mg Hydroxyzin ein Gewichtsverhältnis von Hydroxyzin:Diflunisal von 1:15 bis 1:2,5 entsteht.

26. Synergistische Zusammensetzung nach Anspruch 1, dadurch gekennzeichnet, daß die Einzeldosis-Form 60 bis 300 mg Proquazone enthält und bei einem Gehalt von 25 bis 100 mg Hydroxyzin ein Gewichtsverhältnis von Hydroxyzin:Proquazone von 1:2 bis 1:0,6 entsteht und bei einem Gehalt von 50 bis 100 mg Hydroxyzin ein Gewichtsverhältnis von Hydoxyzin:Proquazone von 1:6 bis 1:0,6 entsteht.

27. Synergistische Zusammensetzung nach Anspruch 26, dadurch gekennzeichnet, daß die Einzeldosis-Form 75 bis 150 mg Proquazone enthält und bei einem Gehalt von 25 bis 100 mg Hydroxyzin ein Gewichtsverhältnis von Hydroxyzin:Proquazone von 1:6 bis 1:0,75 entsteht und bei einem Gehalt von 50 bis 100 mg Hydroxyzin ein Gewichtsverhältnis von Hydroxyzin:Proquazone von 1:3 bis 1:0,75 entsteht.

28. Synergistische Zusammensetzung nach Anspruch 1, dadurch gekennzeichnet, daß die Einzeldosis-Form 200 bis 1300 mg Acetaminophen enthält und bei einem Gehalt von 25 bis 100 mg Hydroxyzin ein Gewichtsverhältnis von Hydroxyzin:Acetaminophen von 1:52 bis 1:2 entsteht und bei einem Gehalt von 50 bis 100 mg Hydroxyzin ein Gewichtsverhältnis von Hydroxyzin:Acetaminophen von 1:26 bis 1:2 entsteht.

29. Synergistische Zusammensetzung nach Anspruch 28, dadurch gekennzeichnet, daß die Einzeldosis-Form 300 bis 650 mg Acetaminophen enthält und bei einem Gehalt von 25 bis 100 mg Hydroxyzin ein Gewichtsverhältnis von Hydroxyzin:Acetaminophen von 1:26 bis 1:2 entsteht und bei einem Gehalt von 50 bis 100 mg Hydroxyzin ein Gewichtsverhältnis von Hydroxyzin:Acetaminophen von 1:13 bis 1:3 entsteht.

30. Synergistische Zusammensetzung nach Anspruch 1, dadurch gekennzeichnet, daß die Einzeldosis-Form 300 bis 800 mg Tolmetin oder ein Salz desselben enthält und bei einem Gehalt von 25 bis 100 mg Hydroxyzin ein Gewichtsverhältnis von Hydroxyzin:Tolmetin oder einem Salz desselben von 1:32 bis 1:3 entsteht und bei einem Gehalt von 50 bis 100 mg Hydroxyzin ein Gewichtsverhältnis von Hydroxyzin:Tolmetin oder einem Salz desselben von 1:16 bis 1:3 entsteht.

31. Synergistische Zusammensetzung nach Anspruch 30, dadurch gekennzeichnet, daß die Einzeldosis-Form 400 bis 600 mg Tolmetin oder ein Salz desselben enthält und bei einem Gehalt von 25 bis 100 mg Hydroxyzin ein Gewichtsverhältnis von Hydroxyzin:Tolmetin oder einem Salz desselben von 1:24 bis 1:4 entsteht und bei einem Gehalt von 50 bis 100 mg Hydroxyzin ein Gewichtsverhältnis von Hydroxyzin:Tolmetin oder einem Salz desselben von 1:12 bis 1:4 entsteht.

**Revendications**

1. Une composition synergique en posologie unitaire pour administration orale ou rectale exerçant un effet analgésique, la composition comprenant:

(a) un ou plusieurs agent(s) anti-inflammatoire(s) non stéroïdiens et/ou acetaminophen; et

(b) de l'hydroxyzine ou un de ses sels pharmaceutiques caractérisée en ce que le composé (a) est un agent anti-inflammatoire non-stéroïdien autre que l'Aspirine et/ou l'acétaminophen et en ce que le contenu du composé (b) est dans la gamme de 25 à 100 mg et est en proportion suffisante par rapport au composé (a) pour réaliser la synergie de l'effet analgésique du composé (a).

2. Une composition synergique suivant la revendication 1 caractérisé en ce que la posologie unitaire contient de 100 mg à 800 mg d'ibuprofen et quand il y a 25 à 100 mg d'hydroxyzine, les rapports de poids hydroxyzine:ibuprofen sont de 1:32 à 1:1 et quand il y a 50 à 100 mg d'hydroxyzine, les rapports de poids hydroxyzine:ibuprofen sont de 1:16 à 1:1.

3. Une composition synergique suivant la revendication 2 caractérisée en ce que la posologie unitaire contient de 150 mg à 600 mg d'ibuprofen et quand il y a 25 à 100 mg d'hydroxyzine, les rapports de poids hydroxyzine:ibuprofen sont de 1:24 à 1:1,5 et quand il y a 50 à 100 mg d'hydroxyzine, les rapports de poids hydroxyzine:ibuprofen sont de 1:12 à 1:1,5.

4. Une composition synergique suivant la revendication 2 ou 3 caractérisée en ce que la posologie unitaire contient de 200 mg à 400 mg d'ibuprofen et quand il y a 25 à 100 mg d'hydroxyzine les rapports de poids hydroxyzine:ibuprofen sont de 1:16 à 1:2 et quand il y a 50 à 100 mg d'hydroxyzine, les rapports de poids hydroxyzine:ibuprofen sont de 1:8 à 1:2.

5. Une composition synergique suivant la revendication 1, caractérisée en ce que la posologie unitaire contient de 300 mg à 1.200 mg d'oxaprozin et quand il y a 25 à 100 mg d'hydroxyzine, les rapports de poids hydroxyzine:oxaprozin sont de 1:48 à 1:3 et quand il y a 50 à 100 mg d'hydroxyzine, les rapports de poids hydroxyzine:oxaprozin sont de 1:24 à 1:3.

6. Une composition synergique suivant la revendication 5 caractérisée en ce que la posologie unitaire contient de 400 mg à 800 mg d'oxaprozin et quand il y a 25 à 100 mg d'hydroxyzine, les rapports de poids

11

**EP 0 111 456 B1**

hydroxyzine:oxaprozin sont de 1:32 à 1:4 et quand il y a 50 à 100 mg d'hydroxyzine, les rapports de poids hydroxyzine:oxaprozin sont de 1:16 à 1:4.

7. Une composition synergique suivant la revendication 1 caractérisée en ce que la posologie unitaire contient de 50 mg à 400 mg de carprofen et quand il y a 25 à 100 mg d'hydroxyzine, les rapports de poids hydroxyzine:carprofen sont de 1:16 à 1:0,5 et quand il y a 50 à 100 mg d'hydroxyzine, les rapports de poids hydroxyzine:carprofen sont de 1:8 à 1:0,5.

8. Une composition synergique suivant la revendication 7 caractérisée en ce que la posologie unitaire contient de 100 mg à 300 mg de carprofen et quand il y a 25 à 100 mg d'hydroxyzine, les rapports de poids hydroxyzine:carprofen sont de 1:12 à 1:1 et quand il y a 50 à 100 mg d'hydroxyzine, les rapports de poids hydoxyzine:carprofen sont de 1:6 à 1:1.

9. Une composition synergique suivant la revendication 1 caractérisée en ce que la posologie unitaire contient de 50 mg à 600 mg de fenoprofen ou un de ses sels et quand il y a 25 à 100 mg d'hydroxyzine, les rapports de poids hydroxyzine:fenoprofen ou d'un de ses sels sont de 1:24 à 1:0,5.

10. Une composition synergique suivant la revendication 9 caractérisée en ce que la posologie unitaire contient de 100 mg à 400 mg de fenoprofen ou d'un de ses sels et quand il y a 25 à 100 mg d'hydroxyzine, les rapports de poids hydroxyzine:fenoprofen ou d'un de ses sels sont de 1:16 à 1:1 et quand il y a 50 à 100 mg d'hydroxyzine, les rapports de poids hydroxyzine:fenoprofen sont de 1:8 à 1:1.

11. Une composition synergique suivant la revendication 9 ou 10 caractérisée en ce que la posologie unitaire contient de 150 mg à 300 mg de fenoprofen ou un de ses sels et quand il y a 25 à 100 mg d'hydroxyzine, les rapports de poids hydoxyzine:fenoprofen ou un de ses sels sont de 1:12 à 1:1,5 et quand il y a 50 à 100 mg d'hydoxyzine, les rapports de poids hydroxyzine:fenoprofen ou un de ses sels sont de 1:6 à 1:1,5.

12. Une composition synergique suivant la revendication 1 caractérisée en ce que la posologie unitaire contient de 100 mg à 600 mg de naproxen ou un de ses sels et quand il y a 25 à 100 mg d'hydroxyzine, les rapports de poids hydoxyzine:naproxen ou un de ses sels sont de 1:24 à 1:1 et quand il y a 50 à 100 mg d'hydroxyzine, les rapports de poids hydroxyzine:naproxen sont de 1:12 à 1:1.

13. Une composition synergique suivant la revendication 12 caractérisée en ce que la posologie unitaire contient de 200 mg à 400 mg de naproxen ou un de ses sels et quand il y a 25 à 100 mg d'hydroxyzine les rapports de poids hydroxyzine:naproxen ou un de ses sels sont de 1:16 à 1:1,2 et quand il y a 50 à 100 mg d'hydroxyzine, les rapports de poids hydroxyzine:naproxen sont de 1:18 à 1:12.

14. Une composition synergique suivant la revendication 1, caractérisée en ce que la posologie unitaire contient de 25 mg à 200 mg d'indoprofen et quand il y a 25 à 100 mg d'hydroxyzine, les rapports de poids hydroxyzine:indoprofen sont de 1:8 à 0,25 et quand il y a 50 à 100 mg d'hydroxyzine, les rapports de poids hydoxyzine:indoprofen sont de 1:4 à 1:1.

15. Une composition synergique suivant la revendication 14 caractérisée en ce que la posologie unitaire contient de 50 mg à 150 mg d'indoprofen et quand il y a 25 à 100 mg d'hydroxyzine, les rapports de poids hydroxyzine:indoprofen sont de 1:6 à 1:0,5 et quand il y a 50 à 100 mg d'hydroxyzine, les rapports de poids hydroxyzine:indoprofen sont de 1:3 à 1:0,5.

16. Une composition synergique suivant la revendication 1 caractérisée en ce que la posologie unitaire contient de 25 mg à 300 mg de ketoprofen et quand il y a 25 à 100 mg d'hydroxyzine, les rapports de poids hydroxyzine:ketoprofen sont de 1:12 à 1:0,25 et quand il y a 50 à 100 mg d'hydroxyzine, les rapports de poids hydroxyzine:ketoprofen sont de 1:6 à 1:0,25.

17. Une composition synergique suivant la revendication 16 caractérisée en ce que la posologie unitaire contient de 50 mg à 200 mg de ketoprofen et quand il y a 25 à 100 mg d'hydroxyzine, les rapports de poids hydroxyzine:ketoprofen sont de 1:8 à 1:0,5 et quand il y a 50 à 100 mg d'hydroxyzine, les rapports de poids hydroxyzine:ketoprofen sont de 1:4 à 1:0,5.

18. Une composition synergique suivant la revendication 1 caractérisée en ce que la posologie unitaire contient de 15 mg à 200 mg de flurbiprofen et quand il y a 25 à 100 mg d'hydroxyzine, les rapports de poids hydroxyzine:flurbiprofen sont de 1:8 à 1:15 et quand il y a 50 à 100 mg d'hydroxyzine, les rapports de poids hydroxyzine:flurbiprofen sont de 1:4 à 1:15.

19. Une composition synergique suivant la revendication 18 caractérisée en ce que la posologie unitaire contient de 25 mg à 150 mg de flurbiprofen et quand il y a 25 à 100 mg d'hydroxyzine, les rapports de poids hydroxyzine:flurbiprofen sont de 1:6 à 1:0,25 et quand il y a 50 à 100 mg d'hydroxyzine, les rapports de poids hydroxyzine:flurbiprofen sont de 1:3 à 1:0,25.

20. Une composition synergique suivant la revendication 1 caractérisée en ce que la posologie unitaire contient de 15 mg à 200 mg de zomepirac et quand il y a 25 à 100 mg d'hydroxyzine, les rapports de poids hydroxyzine:zomepirac sont de 1:4 à 1:0,15 et quand il y a 50 à 100 mg d'hydroxyzine, les rapports de poids hydroxyzine:zomepirac sont de 1:2 à 1:15.

21. Une composition synergique suivant la revendication 20 caractérisée en ce que la posologie unitaire contient de 25 mg à 100 mg de zomepirac et quand il y a 25 à 100 mg d'hydroxyzine, les rapports de poids hydoxyzine:zomepirac sont de 1:4 à 1:0,15 et quand il y a 50 à 100 mg d'hydroxyzine, les rapports de poids hydoxyzine:zomepirac sont de 1:2 à 1:0,25.

22. Une composition synergique suivant la revendication 1 caractérisée en ce que la posologie unitaire contient de 10 mg à 50 mg de piroxicam et quand il y a 25 à 100 mg d'hydroxyzine, les rapports de poids hydroxyzine:piroxicam sont de 1:2 à 1:0,1 et quand il y a 50 à 100 mg d'hydroxyzine, les rapports de poids hydoxyzine:piroxicam sont de 1:1 à 1:0,1.

23. Une composition synergique suivant la revendication 22 caractérisée en ce que la posologie unitaire contient de 15 mg à 37,5 mg de piroxicam et quand il y a 25 à 100 mg d'hydroxyzine, les rapports de poids hydroxyzine:piroxicam sont de 1:1,5 à 1:0,15 et quand il y a 50 à 100 mg d'hydroxyzine, les rapports de poids hydroxyzine:piroxicam sont de 1:0,75 à 1:0,15.

24. Une composition synergique suivant la revendication 1 caractérisée en ce que la posologie unitaire contient de 125 mg à 1.000 mg de diflunisal et quand il y a 25 à 100 mg d'hydroxyzine, les rapports de poids hydroxyzine:diflunisal sont de 1:40 à 1:25 et quand il y a 50 à 100 mg d'hydroxyzine, les rapports de poids hydoxyzine:diflunisal sont de 1:20 à 1:1,25.

25. Une composition synergique suivant la revendication 24, caractérisée en ce que la posologie unitaire contient de 250 mg à 750 mg de diflunisal et quand il y a 25 à 100 mg d'hydroxyzine, les rapports de poids hydroxyzine:diflunisal sont de 1:30 à 1:2,5 et quand il y a 50 à 100 mg d'hydroxyzine, les rapports de poids hydoxyzine:diflunisal sont de 1:15 à 1:2,5.

26. Une composition synergique suivant la revendication 1 caractérisée en ce que la posologie unitaire contient de 60 mg à 300 mg de proquazone et quand il y a 25 à 100 mg d'hydroxyzine, les rapports de poids hydroxyzine:proquazone sont de 1:2 à 1:0,6 et quand il y a 50 à 100 mg d'hydroxyzine, les rapports de poids hydoxyzine:proquazone sont de 1:6 à 1:0,6.

27. Une composition synergique suivant la revendication 26 caractérisée en ce que la posologie unitaire contient de 75 mg à 150 mg de proquazone et quand il y a 25 à 100 mg d'hydroxyzine, les rapports de poids hydroxyzine:proquazone sont de 1:6 à 1:0,75 et quand il y a 50 à 100 mg d'hydroxyzine, les rapports de poids hydroxyzine:proquazone sont de 1:3 à 1:0,75.

28. Une composition synergique suivant la revendication 1 caractérisée en ce que la posologie unitaire contient de 200 mg à 1300 mg d'acetaminofen et quand il y a 25 à 100 mg d'hydroxyzine, les rapports de poids hydroxyzine:acetaminofen sont de 1:52 à 1:2 et quand il y a 50 à 100 mg d'hydroxyzine, les rapports de poids hydoxyzine:acetaminofen sont de 1:26 à 1:2.

29. Une composition synergique suivant la revendication 28 caractérisée en ce que la posologie unitaire contient de 300 mg à 650 mg d'acetaminofen et quand il y a 25 à 100 mg d'hydroxyzine, les rapports de poids hydroxyzine:acetaminofen sont de 1:26 à 1:2 et quand il y a 50 à 100 mg d'hydroxyzine, les rapports de poids hydroxyzine:acetaminofen sont de 1:13 à 1:3.

30. Une composition synergique suivant la revendication 1 caractérisée en ce que la posologie unitaire contient de 300 mg à 800 mg de tolmetin ou un de ses sels et quand il y a 25 à 100 mg d'hydroxyzine, les rapports de poids hydroxyzine:tolmetin sont de 1:8 à 1:0.5 et quand il y a 50 à 100 mg d'hydroxyzine, les rapports de poids hydroxyzine:tolmetin sont de 1:16 à 1:3.

31. Une composition synergique suivant la revendication 30 caractérisée en ce que la posologie unitaire contient de 400 mg à 600 mg de tolmetin ou un de ses sels et quand il y a 25 à 100 mg d'hydroxyzine, les rapports de poids hydroxyzine:tolmetin ou un de ses sels sont de 1:24 à 1:4 et quand il y a 50 à 100 mg d'hydroxyzine, les rapports de poids hydroxyzine:tolmetin ou un de ses sels sont de 1:12 à 1:4.